# EUROPEAN PATENT SPECIFICATION

(11) **EP 4 507 157 B1**
(45) Date of publication and mention of the grant of the patent: **01.10.2025**
(21) Application number: 23191186.8
(22) Date of filing: 11.08.2023
(51) Int. Cl.: H02J 7/00, A61B 5/11, G08B 21/02, G08B 21/22, A61B 5/00

(54) **ELECTRICAL CHARGER FOR AN ELECTRONIC MONITORING BRACELET AND FOR A RECHARGING UNIT OF SAID BRACELET**
ELEKTRISCHE LADEVORRICHTUNG FÜR EIN ELEKTRONISCHES ÜBERWACHUNGSARMBAND UND FÜR EINE NACHFÜLLEINHEIT DES ARMBANDS
CHARGEUR ÉLECTRIQUE POUR BRACELET DE SURVEILLANCE ÉLECTRONIQUE ET POUR UNITÉ DE RECHARGE DUDIT BRACELET

(43) Date of publication of application: 12.02.2025
(73) Proprietor: Geosatis SA, 2340 Le Noirmont (CH)
(72) Inventor: LISON GOMEZ, Alvaro, 2340 Le Noirmont (CH); PRAPLAN, Vincent, 2340 Le Noirmont (CH)
(74) Representative: Bugnion Genève

(56) References cited:
- EP-B1- 2 795 588
- US-A1- 2017 040 825
- US-A1- 2023 081 378
- US-A1- 2023 198 298
- US-B2- 10 020 668
- US-B2- 9 946 297

## Description

### TECHNICAL FIELD AND PRIOR ART

The present invention relates to a charger for an electronic monitoring bracelet and for a recharging unit of said bracelet.

Electronic monitoring bracelet are used for monitoring the displacements of a person, for example within the scope of judicial oversight.

Certain persons suspected of having committed an offence awaiting judgment, or having committed an offence and being on parole, may be forced to wear an electronic monitoring bracelet so that the monitoring authority may localize the person and monitor his/her displacements at any time. Such bracelets contain a satellite localization system (called "GNSS", acronym for Global Navigation Satellite System) such as for example GPS (Global Positioning System), a telecommunications system for transmitting data to a monitoring/control central unit and a system for detecting integrity of the bracelet in order to be able to detect whether the bracelet has been taken off or is faulty.

For example, document EP2795588 describes such an electronic monitoring bracelet. The bracelet as a rigid ring-shaped shell in which the electronic systems are housed.

The internal energy source is provided by rechargeable batteries located inside the casing of the bracelet.

When the internal energy source needs to be recharged, a mobile recharging unit is generally used. The mobile recharging unit has no wire and comprises one or more rechargeable batteries which have been previously recharged by a recharging station or by a USB power supply. When the recharging unit is connected to the bracelet by snap-fitting, power is transmitted to the internal energy source while the person wearing the bracelet can go on moving.

This recharging technology is easy to use and convenient as no wire is required for the recharging step. But if the person wearing the bracelet has not recharged the mobile recharging unit previously, difficulties may arise.

### SUMMARY OF THE INVENTION

It is therefore one goal of the present invention to offer a recharging technology for an electronic monitoring bracelet and/or a recharging unit that is more convenient and easier to use.

The above-recited goal is fulfilled by charger comprising a plug to be connected to a power outlet and a connector, the connector being shaped to be connectable to the bracelet and to the recharging unit.

Thanks to the invention, it is possible to directly recharge the bracelet without using the recharging unit, and the same device is used for recharging the bracelet and the recharging unit.

The direct recharge of the bracelet by the charger according to the invention, which is connected to the electricity grid, can be faster than with the recharging unit.

In an example embodiment, the connector has a curved shape which fits the curved shape of the bracelet shell and a face of the casing of the mobile recharging unit.

The connection between the connector and the bracelet and/or the mobile recharging unit is preferably carried out by magnetic means.

A subject-matter of the invention is then an electrical charger for an electronic monitoring bracelet and a mobile recharging unit for the electronic monitoring bracelet, the electronic monitoring bracelet comprising electrical terminals on one of its faces, and the mobile recharging unit comprising a face with electrical terminals, wherein the charger comprises a plug and a connector connected to each other, the connector comprising a casing with a first face and a second face, the first face comprising electrical terminals and it is configured in such manner that the terminals of the first face cooperate with the terminals of the bracelet and the second face comprises electrical terminals and it is configured in such manner that the terminals of the second face cooperate the terminals of the recharging unit.

Ina preferred embodiment, the electronic monitoring bracelet comprising a ring-shaped shell with electrical terminals on its convex face, and the mobile recharging unit having a concave face with electrical terminals thereon. The first face of the casing of the connector is a concave face and the second face of the casing of the connector is a convex face.

Advantageously, the electrical charger comprises securing means for securing the connector to the bracelet and to the recharging unit. The securing means preferably comprise magnets, the magnets being preferably on the connector.

In an advantageous manner, the electrical charger comprises guiding means to help the connector reach the right position on the bracelet and/or to help the recharging unit reach the right position on the connector.

According to an additional feature, the electrical charger comprises means informing about the status of the recharging step, for example at least one light.

The electrical charger can comprise a wire connected the plug and the connector.

Another subject-matter is a kit comprising an electronic monitoring bracelet, a recharging unit and a charger according to the invention.

The electronic monitoring bracelet can comprise a ring-shaped shell with electrical terminals on its convex face, and the mobile recharging unit has a concave face with electrical terminals on its concave face.

For example, the electronic monitoring bracelet, the recharging unit and the connector of the charger each comprises three electrical terminals.

Preferably, the electronic monitoring bracelet and the recharging unit comprise metallic parts intended to cooperate with the magnets of the connector.

In one example, the guiding means of the connector comprise ridges and/or grooves cooperating with grooves and/or ridges of the electronic monitoring bracelet and/or the recharging unit.

### SHORT DESCRIPTION OF THE DRAWINGS

The present invention will be better understood with the following description and the appended drawings on which:
- Figure 1 is a perspective view of the kit comprising an electronic monitoring bracelet, a mobile recharging unit and a charger according to one example embodiment,
- Figure 2 is a perspective view of the charger connected to the bracelet, on which the upper face of the charger is visible,
- Figure 3 is a perspective view of the charger connected to the mobile recharging unit on which the lower face of the charger is visible,
- Figure 4 is a perspective view of the charger connected to the bracelet,
- Figure 5 is a perspective view of the charger connected to the recharging unit.

### DETAILLED DESCRIPTION OF EMBODIMENTS

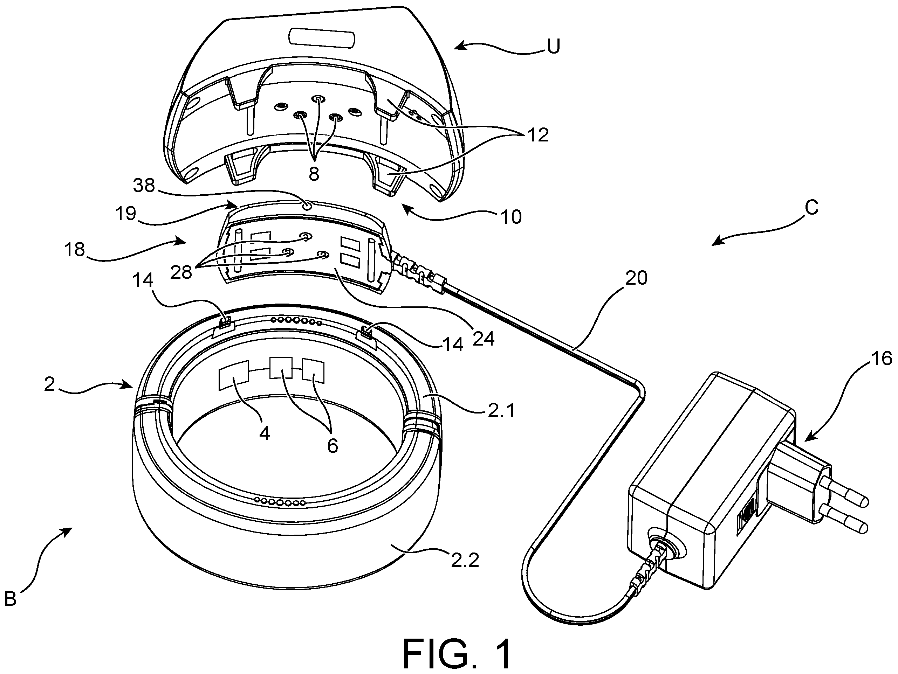

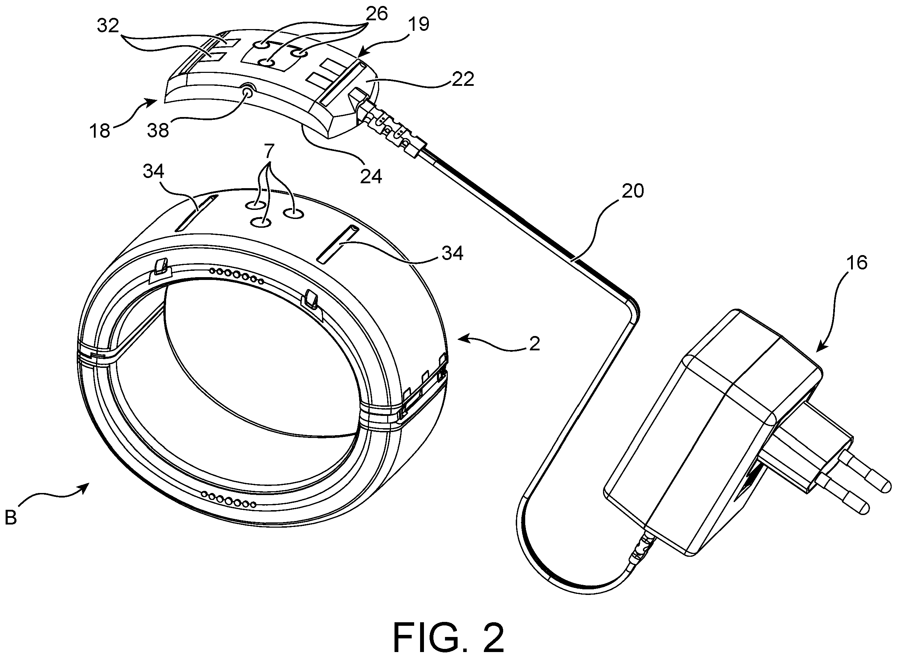

Figures 1, 2 and 4 show an example embodiment of an electronic monitoring bracelet B to which the invention may apply.

In this example embodiment, the electronic monitoring bracelet B, which will be designated by bracelet B in the following description, comprises a rigid ring-shape shell 2 made of several parts 2.1, 2.2, preferably two, which are configured to be rigidly connected to each other in order to surround the wrist or the ankle of a wearer without possibility for the wearer to take off the bracelet by making it slide along the ankle or the wrist. The two parts of the bracelet are half of a circle-shaped attached to each other by locking means, that can be unlocked by specific control operations and are safe enough to prevent the wearer to take off the bracelet without authorization.

Electronic systems 4 (schematically represented) are located inside the shell to localize the wearer and monitor his/her displacements, for example they comprise satellite localization system. In addition, electronic systems comprise means to prevent the wearer to take off the bracelet without this being detected.

The shell of the bracelet is for example made in a rigid plastic material.

The bracelet B will not be described in detail. An example embodiment of such a bracelet is explained in document EP2795588.

In addition, the bracelet comprises one or more rechargeable batteries 6 (schematically represented) inside the shell 2, providing power to the electronic systems 4.

The bracelet B comprises electrical terminals 7, three in the example embodiment of figure 1, on the convex outer face of the shell for the connection to an external recharging system.

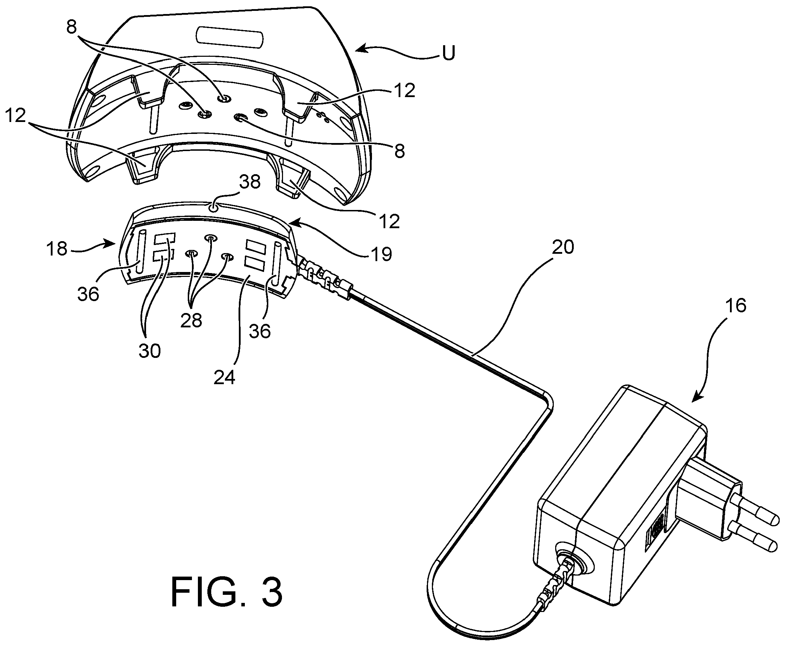

On figures 1 and 3, a mobile recharging unit U is also shown, which comprises rechargeable batteries and is configured to provide power to recharge the rechargeable batteries 6.

The mobile recharging unit U has a shape of a circular arc and the radius of curvature of the recharging unit U corresponds to the one of the bracelet B. When the rechargeable unit U is mounted on the convex outer face of the bracelet B, it contacts a convex outer face of the bracelet.

The concave face of the recharging unit U also comprises three electrical terminals 8 which match the electrical terminals 7 of the bracelet.

Therefore, when the recharging unit U is correctly mounted on the bracelet B, each electrical terminal 8 of the recharging unit contacts an electrical terminal 7 of the bracelet and a recharging phase can be carried out.

The recharging unit U comprises securing means 10 to be secured the bracelet B. In this example, the securing means 10 are snap-fit means and comprises legs 12, for the example of fig.1, which cooperate with protrusions14 on side faces of the bracelet. Any other securing means which ensure a safety attachment of the recharging unit, namely when the wearer moves, can be provided.

The casing of the recharging is for example made in a rigid plastic material similar to the one of the shell of the bracelet.

The recharging unit can be charged by means of USB charger through an USB cable.

According to the invention, a single electrical charger C is provided to allow the direct recharge of the batteries of the bracelet and the batteries of the rechargeable unit as well.

In this example, the charger C comprises a plug 16 configured to be connected to a power outlet (not shown), a connector 18 and a wire 20 between the plug 16 et the connector 18. The plug 16 is able to convert AC into DC. As a variant, the connector is directly connected to the plug.

The connector 18 is configured to be connected to the electrical terminals 7 of the bracelet B and to the electrical terminals 8 of the unit U as well.

The connector 18 comprises a casing 19 having a shape which allow the terminals of the charger to contact the terminals of the bracelet and the terminals of the recharging unit.

In the example shown, the casing has a circle arc shape with a radius of curvature corresponding to the one of the bracelet and to the one of the recharging unit U.

The casing of the connector is for example made in a rigid plastic material similar to the one of the shell of the bracelet and of the casing of the recharging unit.

The connector 18 then comprises a convex face 22 intended to contact the concave face of the recharging unit U and a concave face 24 intended to contact the convex face of the bracelet B.

The connector 18 comprises terminals 26 on its convex face 22 and terminals 28 in its concave face 24 intended to cooperate with electrical terminals of the bracelet and the terminals of the recharging unit respectively.

In a preferred embodiment, securing means are provided between the connector 18 and the bracelet B, and between the connector 18 and the recharging unit U.

In a very advantageous manner, securing means are magnetic means which render the connection easy and fast. The concave and convex faces of the connector 18 comprise magnets 30, 32 respectively which cooperates with metallic parts of the bracelet and of the recharging unit U, or any other material able to be attracted by magnets. As a variant the magnets are on the bracelet and the recharging unit. As another variant, one face of the connector comprises magnets and the other face comprises metallic parts.

Preferably, the connector comprises guiding means to ensure thar the connector has the right position on the bracelet in which the terminals are in contact with each other. In the example shown on figures, the guiding means comprise grooves 34 on the convex face of the bracelet and parallel to the axis of the bracelet, and correspond ridges 36 on the concave face of the connector 18. Advantageously, guiding means are also provided between the connector 18 and the recharging unit U, which are similar to the guiding means between the connector and the bracelet. Any other guiding means may be provided to help the wearer to connect the charge to the bracelet and/or the recharging unit. Grooves and ridges can be exchanged.

As a variant, mechanical securing means instead of magnetic securing means are provided between the connector 18 and the bracelet B and/or between the connector 18 and the recharging unit U. For example, the legs of the snap-fitting means of the recharging unit may be used to secure the recharging unit to the connector.

In addition, the connector advantageously comprises at a least a light 38 for example a LED (light-emitting diode) for informing the wearer of the status of the recharging step, for example in process or completed. The colour of the light can change depending on the status, and/or the light can be a flashing light or a continuous light depending on the charge level of the batteries. The light can also inform the wearer when the connector is not correctly in position on the bracelet or on the recharging unit.

An example of the use of the invention will now be described.

The batteries of the bracelet need to be recharged. A warning signal may be emitted, for example a warning sound.

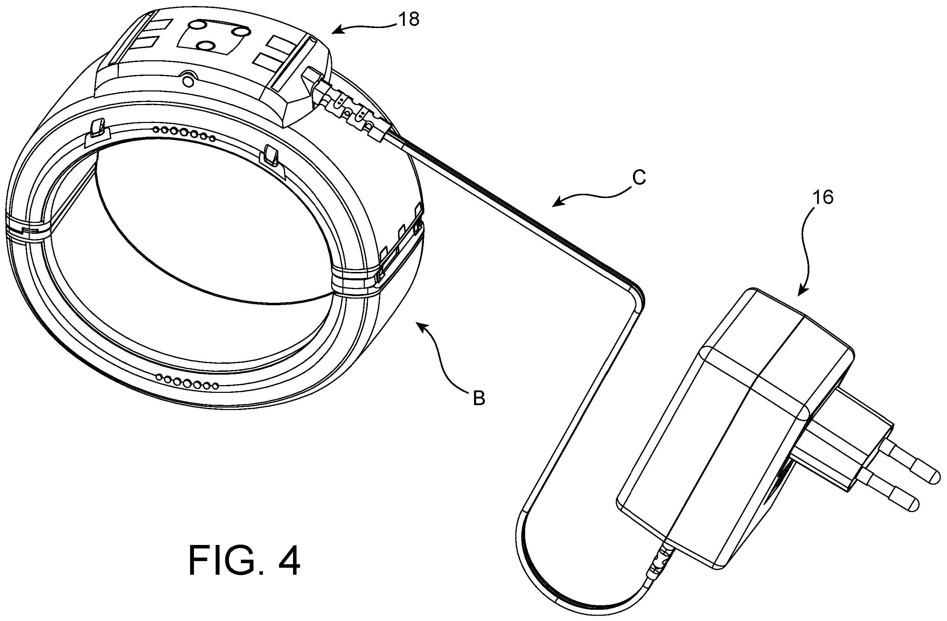

The wearer is then informed that he must recharge the bracelet batteries. He or she has a recharging unit U, he or she can use the recharging unit U, but if it has not been previously recharged, he or she uses the charger C. He or she puts in place the connector 18 on the convex face of the bracelet B in such manner that the terminals of the connector 18 match the terminals 7 of the bracelet. The wearer is helped by the guiding means and the magnetic securing means which fix the position of the connector on the bracelet. Figure 4 shows the charger connected to the bracelet.

The wearer then plugs in the charger and the recharging step begins. The colour of the light and/or its illuminous state inform the wearer that the recharging step is in progress. When the recharging step is finished, the wearer is also informed by a change in the light appearance. As a variant sound signals can be emitted.

During the recharging step of the bracelet by the charger, the freedom to move of the wearer is limited by the length of the wire of the charger.

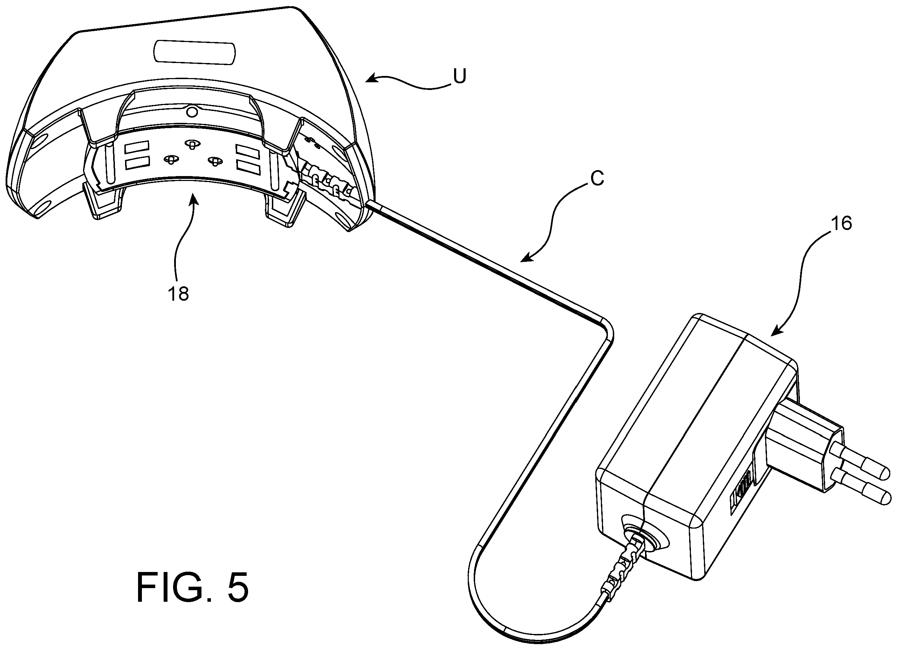

The wearer can also recharge the recharging unit in order that it is ready to be used for recharging the bracelet in a mobile way. In this case, the wearer puts the recharging unit U on the connector 18 aligning the connector terminals with the recharging unit terminals, helped by the positioning means and the magnetic securing means. In the similar manner as described for the recharging step of the bracelet, the light of the connector changes depending on the charging status of the batteries. Figure 5 shows the charger connected to the recharging unit.

In another example, the connector can be used to simultaneously charge the bracelet and the recharging unit, the connector being in contact with the bracelet by its concave face and with the recharging unit by its convex face.

The connector may be used to unlock the bracelet.

In the example of figures 1 to 3, the width of the connector is smaller than the width of the shell of bracelet and of the casing of the recharging unit, but a connector having the same width as the shell and the casing belongs to the invention.

The ring-shape of the bracelet with convex face, and the corresponding shape of the recharging unit are not limiting. In another example, the outer face of the bracelet is polygonal. The shape of the connector is then adapted to this shape, and can be flat or comprise two faces connected by an angle. Any other shape suitable for the bracelet, the recharging unit and the connector does not depart from the scope of the invention.

Thanks to the invention, the charging station may be omitted and the wearer only uses the charger of the invention. In addition, the charger is less cumbersome than a charging station and requires less material.

Furthermore, the wearer can recharge the bracelet even if the recharging unit is not ready for recharging the bracelet.

## Claims

1. Electrical charger for an electronic monitoring bracelet (B) and a mobile recharging unit (U) for the electronic monitoring bracelet (C), the electronic monitoring bracelet (B) comprising electrical terminals (7) on one of its faces, and the mobile recharging unit (U) comprising a face with electrical terminals (8), wherein the charger comprises a plug (16) and a connector (18) connected to each other, the connector (18) comprising a casing (19) with a first face (24) and a second face (22), the first face (24) comprising electrical terminals (28) and it is configured in such manner that the terminals of the first face cooperate with the terminals (7) of the bracelet (B) and the second face (22) comprises electrical terminals (26) and it is configured in such manner that the terminals of the second face cooperate the terminals (8) of the recharging unit (U).

2. Electrical charger according to claim 1, in which the electronic monitoring bracelet (C) comprising a ring-shaped shell (2) with electrical terminals (7) on its convex face, and the mobile recharging unit (U) having a concave face with electrical terminals (8) thereon, and in which the first face (24) of the casing (19) of the connector (18) is a concave face (24) and the second face (22) of the casing of the connector (18) is a convex face (22).

3. Electrical charger according to claim 1 or 2, comprising securing means for securing the connector to the bracelet and to the recharging unit.

4. Electrical charger according to claim 3, in which the securing means comprise magnets (30, 32), the magnets being preferably on the connector (18).

5. Electrical charger according to any of the claims 1 to 4, comprising guiding means to help the connector reach the right position on the bracelet and/or to help the recharging unit reach the right position on the connector.

6. Electrical charger according to any of the claims 1 to 5, comprising means informing about the status of the recharging step, for example at least one light (38).

7. Electrical charger according to one of the preceding claims, comprising a wire (20) connected the plug (16) and the connector (18).

8. Kit comprising an electronic monitoring bracelet (B), a recharging unit (U) and a charger (C) according to one of the preceding claims.

9. Kit according to claim 8, in which the electronic monitoring bracelet (B) comprises a ring-shaped shell (2) with electrical terminals (7) on its convex face, and the mobile recharging unit (U) has a concave face with electrical terminals (8) on its concave face.

10. Kit according to the preceding claim, in which the electronic monitoring bracelet (B), the recharging unit (U) and the connector (18) of the charger (C) each comprises three electrical terminals.

11. Kit according to claim 8, 9 or 10 in combination with claim 4, in which the electronic monitoring bracelet and the recharging unit comprise metallic parts intended to cooperate with the magnets of the connector.

12. Kit according to any of the claims 8 to 11, in combination with claim 5, in which the guiding means of the connector comprise ridges (36) and/or grooves cooperating with grooves (32) and/or ridges of the electronic monitoring bracelet and/or the recharging unit.

## Patentansprüche

1. Elektrische Ladevorrichtung für ein elektronisches Überwachungsarmband (B) und mobile Wiederaufladeeinheit (U) für das elektronische Überwachungsarmband (C), wobei das elektronische Überwachungsarmband (B) elektrische Klemmstellen (7) auf einer seiner Seiten umfasst, und wobei die mobile Wiederaufladeeinheit (U) eine Seite mit elektrischen Klemmstellen (8) umfasst, wobei die Ladevorrichtung einen Stecker (16) und einen Verbinder (18) umfasst, die miteinander verbunden sind, wobei der Verbinder (18) ein Gehäuse (19) mit einer ersten Seite (24) und einer zweiten Seite (22) umfasst, wobei die erste Seite (24) elektrische Klemmstellen (28) umfasst und derart konfiguriert ist, dass die Klemmstellen der ersten Seite mit den Klemmstellen (7) des Armbands (B) zusammenarbeiten und die zweite Seite (22) elektrische Klemmstellen (26) umfasst und derart konfiguriert ist, dass die Klemmstellen der zweiten Seite mit den Klemmstellen (8) der Wiederaufladeeinheit (U) zusammenarbeiten.

2. Elektrische Ladevorrichtung nach Anspruch 1, wobei das elektronische Überwachungsarmband (C) eine ringförmige Schale (2) mit elektrischen Endklemmen (7) auf seiner konvexen Seite umfasst, und wobei die mobile Wiederaufladeeinheit (U) eine konkave Seite mit elektrischen Endklemmen (8) darauf aufweist, und wobei die erste Seite (24) des Gehäuses (19) des Verbinders (18) ein konkave Seite (24) ist und die zweite Seite (22) des Gehäuses des Verbinders (18) ein konvexe Seite (22) ist.

3. Elektrische Ladevorrichtung nach Anspruch 1 oder 2, umfassend Befestigungsmittel zur Befestigung des Verbinders auf dem Armband und mit der Wiederaufladeeinheit.

4. Elektrische Ladevorrichtung nach Anspruch 3, wobei die Sicherungsmittel Magnete (30, 32) umfassen, wobei die Magnete vorzugsweise auf dem Verbinder (18) sind.

5. Elektrische Ladevorrichtung nach einem der Ansprüche 1 bis 4, umfassend Führungsmittel, um den Verbinder dabei zu unterstützen, die korrekte Position auf dem Armband zu erreichen, und/oder die Wiederaufladeeinheit dabei zu unterstützen, die korrekte Position auf dem Verbinder zu erreichen.

6. Elektrische Ladevorrichtung nach einem der Ansprüche 1 bis 5, umfassend Mittel, die über den Status des Wiederaufladeschritts informieren, z. B. mindestens ein Licht (38).

7. Elektrische Ladevorrichtung nach einem der vorhergehenden Ansprüche, umfassend einen Draht (20), der mit dem Stecker (16) und dem Verbinder (18) verbunden ist.

8. Kit, umfassend ein elektronisches Überwachungsarmband (B), eine Wiederaufladeeinheit (U) und eine Ladevorrichtung (C) nach einem der vorhergehenden Ansprüche.

9. Kit nach Anspruch 8, wobei das elektronische Überwachungsarmband (B) eine ringförmige Schale (2) mit elektrischen Endklemmen (7) auf ihrer konvexen Seite umfasst und wobei die mobile Wiederaufladeeinheit (U) eine konkave Seite mit elektrischen Endklemmen (8) auf ihrer konkaven Seite umfasst.

10. Kit nach dem vorhergehenden Anspruch, wobei das elektronische Überwachungsarmband (B) die mobile Wiederaufladeeinheit (U) und der Verbinder (18) der Ladevorrichtung (C) jeweils drei elektrische Endklemmen umfassen.

11. Kit nach Anspruch 8, 9 oder 10 in Kombination mit Anspruch 4, wobei das elektronische Überwachungsarmband und die Wiederaufladeeinheit metallische Teile umfassen, die ausgelegt sind, um mit den Magneten des Verbinders zusammenzuarbeiten.

12. Kit nach einem der Ansprüche 8 bis 11 in Kombination mit Anspruch 5, wobei die Führungsmittel, des Verbinders Rippen (36) und/oder Nuten umfassen die mit den Nuten (32) und/oder Rippen des elektronischen Überwachungsarmbands und/oder die Wiederaufladeeinheit zusammenarbeiten.

## Revendications

1. Chargeur électrique pour bracelet de surveillance électronique et une unité de recharge mobile (U) pour le bracelet de surveillance électronique (C), le bracelet de surveillance électronique (B) comprenant des bornes électriques (7) sur l'une des ses faces, et l'unité de recharge mobile (U) comprenant une face avec des bornes électriques (8), dans lequel le chargeur comporte une prise (16) et un connecteur (18) connectés l'un à l'autre, le connecteur (18) comprenant un boîtier (19) muni d'une première face (24) et d'une seconde face (22), la première face (24) comprenant des bornes électriques (28) et est configurée de sorte que les bornes de la première face coopèrent avec les bornes (7) du bracelet (B) et la seconde face (22) comporte des bornes électriques (26) et est configurée de sorte que les bornes de la seconde face coopèrent avec les bornes de l'unité de recharge (U).

2. Chargeur électrique selon la revendication 1, dans lequel bracelet de surveillance électronique (C) comporte un coque en forme d'anneau (2) munie des bornes électriques (7) sur sa face convexe, et l'unité de recharge mobile (U) ayant une face concave des bornes électriques (8) sur celle-ci, et dans lequel la première face (24) du boîtier (19) du connecteur (18) est une face concave et la seconde face (22) du boîtier du connecteur (18) est une face convexe.

3. Chargeur électrique selon la revendication 1 ou 2, comprenant des moyens de fixation pour fixer le connecteur au bracelet et à l'unité de recharge.

4. Chargeur électrique selon la revendication 3, dans lequel les moyens de fixation comportent des aimants (30, 32), les aimants étant de préférence sur le connecteur (8).

5. Chargeur électrique selon l'une des revendications 1 à 4, comportant des moyens de guidage pour aider le connecteur à atteindre la position correcte sur le bracelet et/ou aider l'unité de recharge à atteindre la position correcte sur le connecteur.

6. Chargeur électrique selon l'une des revendications 1 à 5, comprenant des moyens informant sur l'état de l'étape de recharge, par exemple au moins un voyant lumineux (38).

7. Chargeur électrique selon l'une des revendications précédentes, comprenant un câble (20) connectant la prise (16) et le connecteur (18).

8. Kit comprenant un bracelet de surveillance électronique (B), une unité de recharge (U) et un chargeur selon l'une des revendications précédentes.

9. Kit selon la revendication 8, dans lequel bracelet de surveillance électronique (B) comporte une coque en forme d'anneau (2) avec des bornes électriques (7) sur sa face convexe, et l'unité de recharge mobile comporte une face concave avec des bornes électriques (8) sur sa face concave.

10. Kit selon la revendication précédente, dans lequel le bracelet de surveillance électronique (B), l'unité de recharge (U) et le connecteur (18 ) du chargeur (C) comportent chacun trois bornes électriques.

11. Kit selon la revendication 8, 9 ou 10 en combinaison avec la revendication 4, dans lequel le bracelet de surveillance électronique (B) et l'unité de recharge comportent des parties métalliques destinées à coopérer avec les aimants du connecteur.

12. Kit selon l'une des revendications 8 à 11, en combinaison avec la revendication 5, dans lequel les moyens de guidage du connecteur comportent des nervures (36) et/ou des rainures coopérant avec des rainures (32) et/ou des nervures du bracelet de surveillance électronique (B) et/ou de l'unité de recharge.
